# EUROPEAN PATENT APPLICATION

(11) **EP 4 095 227 A1**
(43) Date of publication of application: **30.11.2022**
(21) Application number: 20915934.2
(22) Date of filing: 18.09.2020
(51) Int. Cl.: C12M 1/34, C12M 1/33, C12M 1/00

(54) **CELL-EXTRUDING DEVICE INCLUDING MEMBRANE MODULE, AND CELL-EXTRUDING METHOD USING SAME**

(30) Priority: 22.01.2020 KR 20200008298; 22.01.2020 KR 20200079466
(71) Applicant: MDimune Inc., Seoul 04790 (KR)
(72) Inventor: HAN, Dongwoo, Suwon-si, Gyeonggi-do 16699 (KR); BAE, Shingyu, Seongnam-si, Gyeonggi-do 13547 (KR); OH, Seungwook, Suwon-si, Gyeonggi-do 16435 (KR); SIM, Yunbeom, Seoul 02598 (KR); LEE, Seonjoo, Seoul 08741 (KR)
(74) Representative: Dehns
(86) International application number: PCT/KR2020/012628
(87) International publication number: WO 2021/149891

(57) **Abstract**

The present invention relates to a cell-extruding device including a membrane module, and a cell-extruding method using same, and more particularly, to a cell-extruding device, and a cell-extruding method using same, wherein a suspension including cells is efficiently dispersed in the membrane module, thus preventing membrane clogging and increasing the usable area of the membrane, and replacing the membrane and carrying out the process are easy, thus increasing production efficiency.

## Description

### [Technical Field]

The present invention relates to a cell extrusion device including a membrane module and a cell extrusion method using the same, and more particularly, to a cell extrusion device, which can prevent a membrane clogging phenomenon and increase a usable area of a membrane by effectively dispersing and extruding a cell-containing suspension in a membrane module, and can increase production efficiency by replacing a membrane and performing a process easily, and a cell extrusion method using the same.

### [Background Art]

Extracellular vesicles refer to various types of particles secreted from cells, and may be broadly classified into exosomes derived from an endosomal pathway and microvesicles derived from a plasma membrane.

Exosomes, which are spherical vesicles discharged by cells, contain various types of information such as proteins and DNA of a parent cell, so they can be used as a biomarker to actively perform the development of cancer diagnosis markers and sensors.

In addition, microvesicles are generally a type of cell organelles with a size of 0.03 to 1 µm, naturally released from the cell membrane of a cell, having a phospholipid bilayer, and containing components in the cytoplasm, such as mRNA, DNA, and a protein.

Recently, research on drug delivery systems using these extracellular vesicles is being actively conducted, and a small amount of a drug can be encapsulated in a vesicle to deliver the corresponding drug to a specific site, so the applicability of these drug delivery systems is growing in various medical fields using extracellular vesicles. Particularly, since microvesicles are directly released from the cell membrane, they retain an antigen of a parent cell, so the applicability of vaccines is very high.

However, since the quantity of extracellular vesicles that can be obtained from cells is very limited, recently, a method of manufacturing extracellular vesicle mimetics for artificially obtaining a large amount is required.

Conventionally, a centrifuge can be used as a method for obtaining extracellular vesicle mimetics, but have limitations in terms of high prices and a considerably low yield that still falls short of expectations.

### [Disclosure]

### [Technical Problem]

To solve the above problems, the present invention is directed to providing a cell extrusion device which can increase the usable area of a membrane in an extrusion process by increasing the dispersion rate of a suspension by applying a dispersion disk that efficiently disperses the suspension including cells in a membrane module, and increase production efficiency by enabling mass-production by using a syringe with a large volume, and a cell extrusion method using the same.

### [Technical Solution]

To achieve the above purposes, the present invention provides a cell extrusion device for extruding cells in a suspension passing through a membrane module through constant pressure, wherein the membrane module includes a pair of supports having a through-hole through which the suspension flows; a membrane through which the cells pass and are extruded; and a dispersion disk having a plurality of micro holes except a dispersing zone having a center. The membrane and the dispersion disk are located between the pair of supports, the through-holes respectively formed in the pair of supports and the center of the dispersion disk are located on the same axis, and the membrane module is closely fixed by a pair of holders.

The membrane module has an O-ring between the dispersion disk and a support adjacent to the dispersion disk, thereby forming a predetermined sealed space, and according to this, the suspension passing through the support may be diffused into a number of micro holes formed in the dispersion disk.

In addition, by forming a sealed space using an O-ring located between the dispersion disk and the membrane, the membrane permeation efficiency of the suspension may be increased.

When the membrane module has a porous membrane supporting film on one or more surfaces of the membrane, damage to the membrane may be prevented.

The present invention also provides a cell extrusion method using the cell extrusion device, which includes connecting a syringe having a cell-containing suspension to a through-hole of a support adjacent to the dispersion disk among the through-holes of the cell extrusion device; and injecting the suspension contained in the syringe into the membrane module and discharging the suspension through the other through-hole via the dispersion disk and the membrane, the entire process of which is repeated at least once.

The present invention also provides a cell extrusion device for extruding cells in a suspension passing through a membrane module through a predetermined pressure, which includes a pair of supports. The membrane module includes a pair of supports having a through-hole through which the suspension flows; a membrane through which the cells pass and are extruded; and a dispersion disk having a plurality of micro holes except a dispersing zone having a center. The membrane and the dispersion disk are located between the pair of supports, the through-holes respectively formed in the pair of supports and the center of the dispersion disk are located on the same axis, and the membrane module is closely fixed by a pair of holders.

The membrane module has a sealed space formed by an O-ring between the support and the dispersion disk, such that a suspension passing through the support can be diffused to a plurality of micro holes formed in the dispersion disk.

In addition, the membrane module may increase the membrane permeation efficiency of the suspension by forming a predetermined sealed space by an O-ring located between the membrane and the dispersion disk.

In addition, the membrane module may have a porous membrane supporting film on one or more surfaces of the membrane, thereby preventing the membrane from being damaged.

In the membrane module, a syringe including the suspension may be connected to any one or more of the through-holes formed in the pair of supports, and in the syringe, a plunger may move by means of a syringe pump.

The present invention also provides a cell extrusion method using the cell extrusion device of the present invention, which includes:
connecting a syringe including a cell-containing suspension to a through-hole of a support adjacent to the dispersion disk among the through-holes of the cell extrusion device; and
injecting the suspension in the syringe into the membrane module and then discharging the suspension through the other through-hole via the dispersion disk and the membrane, and
the entire process of which is repeated at least once.

The present invention also provides a cell extrusion method using the cell extrusion device, which includes: connecting a first syringe including a cell-containing suspension to a through-hole at one side of the cell extrusion device, and connecting a second syringe in which a plunger has been pressed to a through-hole at the other side of the cell extrusion device; injecting the suspension contained in the first syringe into the membrane module to allow the suspension to flow into the second syringe through the dispersion disk and the membrane; and injecting the suspension flowing into the second syringe into the membrane module again to allow the suspension to flow into the first syringe through the dispersion disk and the membrane, and the entire process of which is repeated at least once.

### [Advantageous Effects]

The present invention has the following effects:
1. Since a large-capacity syringe can be used to inject a cell-containing suspension into a membrane module, it is possible to be easily manipulated and rapidly extrude the cells.
2. Since through-holes formed in a pair of supports located in a membrane module and the center of a dispersion disk are located on the same axis, and micro holes are not formed in a dispersion zone of the dispersion disk, a suspension passing through the through-holes is dispersed while colliding with the dispersion zone of the dispersion disk, so that the suspension can pass through the micro holes open to all directions. Therefore, not only the usable area of a membrane can be increased, but also membrane clogging occurring during repeated use can be prevented.
3. When extrusion is performed by connecting syringes to both ends of an extrusion device having a pair of dispersion disks located at both sides based on a membrane in a membrane module, a continuous extrusion process can be performed to shorten extrusion time.
4. Since a close contact state is fixed or released by a pair of holders to which a membrane module is mutually fastened, membranes with different pore sizes can be easily replaced, and when a disposable product is used for all internal components of the membrane module, the possibility of contamination during the process can be reduced.

### [Description of Drawings]

FIG. 1 is an exploded perspective view of a cell extrusion device according to a first embodiment of the present invention.
FIG. 2 is an exploded cross-sectional view of the cell extrusion device according to the first embodiment of the present invention.
FIG. 3 is a cross-sectional view of a dispersion disk according to the present invention.
FIG. 4 is a perspective view of a syringe connected to one side of the cell extrusion device according to the first embodiment of the present invention.
FIG. 5 is an exploded perspective view of a cell extrusion device according to a second embodiment of the present invention.
FIG. 6 is an exploded cross-sectional view of the cell extrusion device according to the second embodiment of the present invention.
FIG. 7 is a perspective view of syringes connected to both sides of the cell extrusion device according to the second embodiment of the present invention.
FIG. 8 is a cross-sectional view showing that the syringes connected to both sides of the cell extrusion device according to the second embodiment of the present invention move by using syringe pumps.

### [Modes of the Invention]

Hereinafter, embodiments according to the present invention will be described in detail with reference to the accompanying drawings.

Various embodiments of the present invention are illustrated by drawings, and will be described in detail below. However, it is not intended to limit the present invention to the particular forms disclosed, and rather, the present invention includes all modifications, equivalents and substitutions consistent with the spirit of the present invention as defined by the claims. However, in the accompanying drawings, thicknesses and sizes are exaggerated for clarity of the specification, and therefore, the present invention is not limited by the relative size or thickness shown in the accompanying drawings.

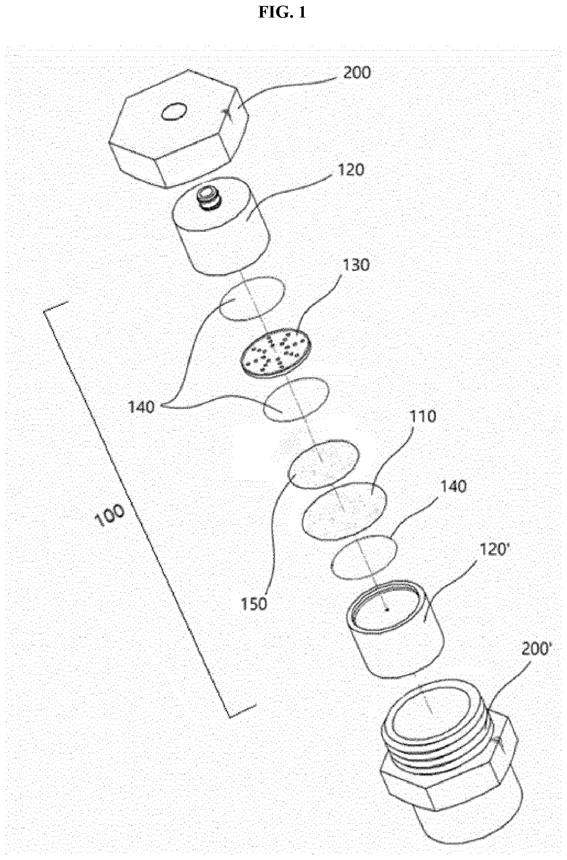

Referring to FIGS. 1 to 4, a cell extrusion device (hereinafter, referred to as an "extrusion device") according to one embodiment of the present invention will be described.

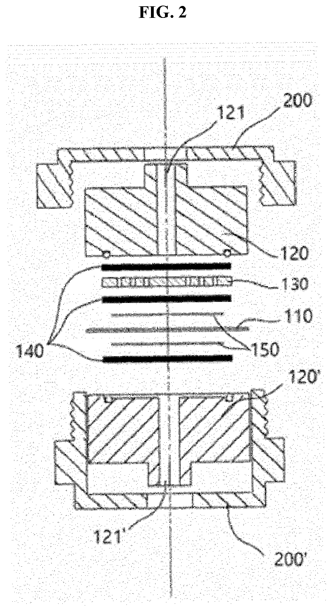

FIG. 1 is an exploded perspective view of an extrusion device 10 according to a first embodiment of the present invention, and FIG. 2 is an exploded cross-sectional view of the extrusion device 10 according to the first embodiment of the present invention.

The extrusion device 10 includes a membrane module 100 and a pair of holders 200 and 200' closely fixed to the membrane module 100.

The membrane module 100 extrudes cells when a cell-containing suspension 300 passes through a membrane 110 with fine porosity while passing through the membrane module 100.

The cell-containing suspension 300 may be injected into the membrane module 100 using a syringe. However, the injection means of the suspension 300 is not necessarily limited to a syringe, and it is fine as long as the suspension 300 can be pressurized in a predetermined direction and discharged.

The membrane module 100 includes a pair of supports 120 and 120' and a membrane 110 and a dispersion disk 130 between the pair of supports 120 and 120'.

The supports 120 and 120' are respectively formed with through-holes 121 and 121' through which the suspension flows. The through-holes 121 and 121' are preferably formed in the center of the supports 120 and 120' to make the flow axis of the suspension 300 linear.

The ends of the through-holes 121 and 121' may protrude to a predetermined height from the supports 120 and 120' for easy coupling with the end of a syringe 400 including the cell-containing suspension 300. The ends of the supports 120 and 120' may protrude to a predetermined height for easy coupling with the end of the syringe 400. If, at the end of the syringe 400, a screw thread can be formed for coupling with a needle, in this case, when the ends of the supports 120 and 120' have screw threads corresponding to the screw thread formed at the end of the syringe 400, it can make the coupling with the syringe 400 tighter.

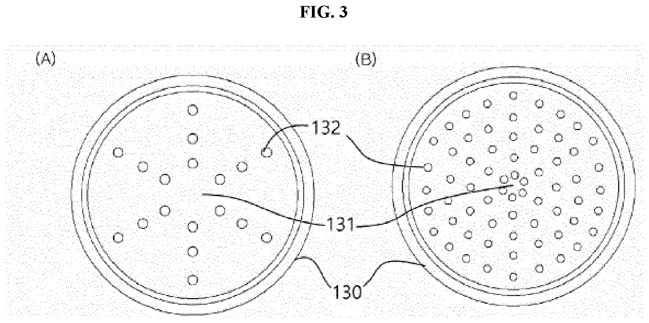

Referring to FIGS. 3A and 3B, the dispersion disk 130 has a plurality of micro holes 132 except a dispersion zone 131 including a center, and the injected suspension 300 moves at a high speed along through-holes 121 and is widely dispersed while colliding with the dispersion zone 131 of the dispersion disk 130.

Here, to increase dispersion efficiency, the through-holes 121 and 121' formed in the supports 120 and 120' located in the membrane module 100 and the center of the dispersion disk 130 are located on the same axis.

The dispersion disk 130 and an O-ring 140 between the dispersion disk 130 and the adjacent support 120 are preferably located such that the suspension 300 dispersed by the dispersion disk 130 receives a continuous pressure and passes through a plurality of micro holes 132 formed in the dispersion disk 130 without flowing out to the outside. This is because, when the O-ring 140 is in the above position, a sealed space is formed between the dispersion disk 130 and the support 120 while being in a close contact state, and through this, the dispersion effect of the suspension 300 may be further increased.

The membrane module 100 may further have an O-ring 140 between the dispersion disk 130 and the membrane 110, thereby forming a sealed space in a close contact state, so a continuous pressurization state may be maintained when the suspension 300 passes through the membrane 300.

In addition, to increase the sealed close contact between the components of the membrane module 100 as necessary, an additional O-ring 140 may be further located.

In addition, in the membrane module 100, a membrane supporting film 150 with porosity may be further located on at least one surface of the membrane 110. The membrane supporting film 150 prevents the membrane 110 from being damaged when a predetermined pressure is applied to the membrane 110. A pore size of the membrane supporting film 150 must be greater than that of the membrane 110 to facilitate the movement of the suspension 300.

In the membrane module 100, internal components may be in a close contact state with each other, so the suspension 300 may smoothly pass through the membrane 110 in a predetermined pressurized state without leakage of the suspension 300. Accordingly, for close contact with the membrane module 100, the pair of holders 200 and 200' accommodate the membrane module 100 and are closely fixed by a fastening means. The fastening means may be a screw thread corresponding to each other as shown in FIGS. 1 and 2, and may be, but is not limited to, any of the conventional fastening means. At the ends of the pair of holders 200 and 200', predetermined holes are formed to expose the ends of the supports 120 and 120'. A material for the holders 200 and 200' may be, but is not particularly limited to, a metal in order to withstand a pressure caused by constant close contact.

In the cell extrusion process, the replacement of the membrane 110 having a different pore size occurs frequently, and the holders 200 and 200' may release the close contact state with a simple operation, so they have an advantage of facilitating the replacement of the membrane 110.

In addition, in the cell extrusion process, since it is important to clean the membrane module 100, it is preferable to use disposable materials with low unit prices as the components of the membrane module 100 according to the present invention.

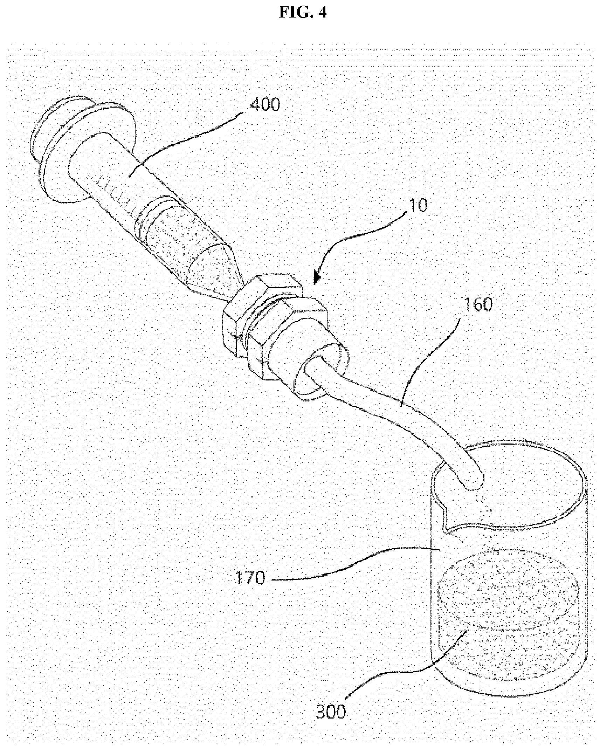

Hereinafter, referring to FIG. 4, a cell extrusion method using the extrusion device 10 according to one embodiment of the present invention will be described.

Since the extrusion device 10 has one dispersion disk 130, it is preferable that the injection direction of the suspension 300 is also unidirectional. Accordingly, as shown in FIG. 4, a syringe 400 containing a cell-containing suspension 300 is connected to one side of the extrusion device 10, and a connection hose 160 guiding a suspension discharged through the membrane 110 to the outside is connected to the opposite side such that the suspension 300 is accommodated in a container 170 for accommodating the suspension 300.

First, the syringe 400 containing the cell-containing suspension 300 is connected to a through-hole 121 formed in a support 120 adjacent to the dispersion disk 130 among through-holes 121 and 121' of the cell extrusion device 10 (S10)

Subsequently, when a plunger inside the syringe 400 is moved to inject the suspension 300 contained in the syringe 400 into the membrane module, the suspension 300 is discharged by the other through-hole 121' through the dispersion disk 130 and the membrane 110 (S20).

S10 and S20 may be repeated at least once, and more preferably, 3 to 5 times, to obtain a sufficient extrusion effect.

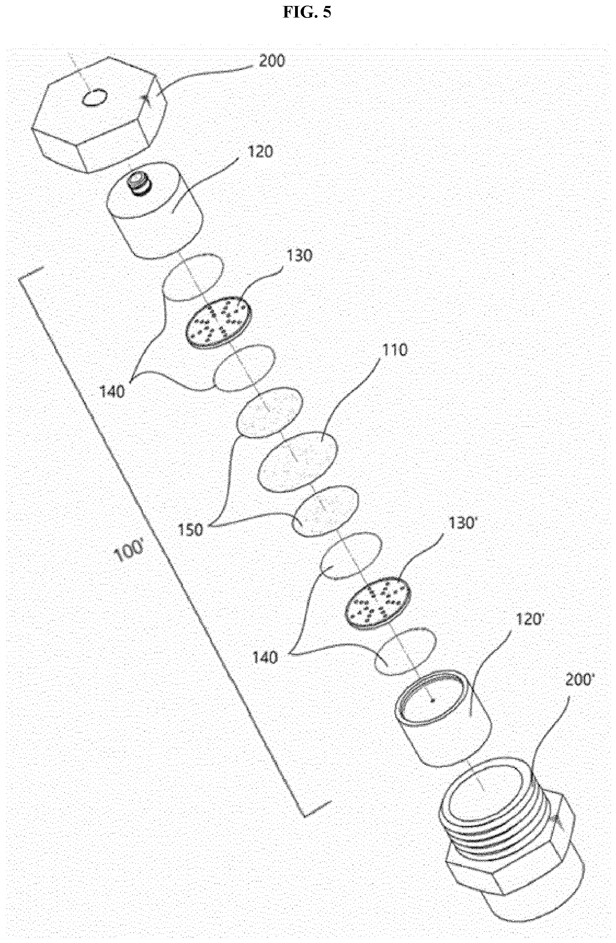

Next, an extrusion device 10' according to a second embodiment of the present invention will be described with reference to FIGS. 5 to 8.

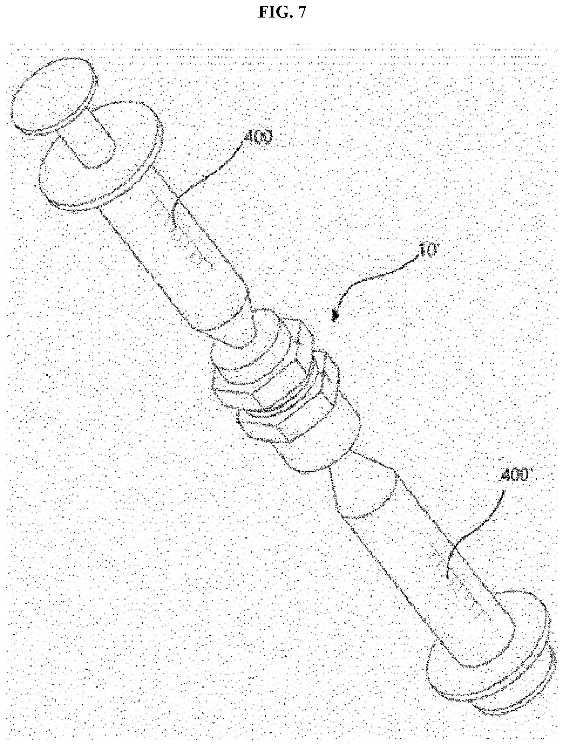

The extrusion device 10' according to a second embodiment of the present invention is overall similar to the extrusion device 10 according to the first embodiment of the present invention, except that a pair of dispersion disks 130 and 130' are located, and the shapes and functions of a membrane 110, supports 120 and 120', and dispersion disks 130 and 130' are the same as described above. Accordingly, in the extrusion device 10' according to the second embodiment, as shown in FIG. 7, two syringes 400 and 400' are connected to both ends to facilitate a bidirectional extrusion process.

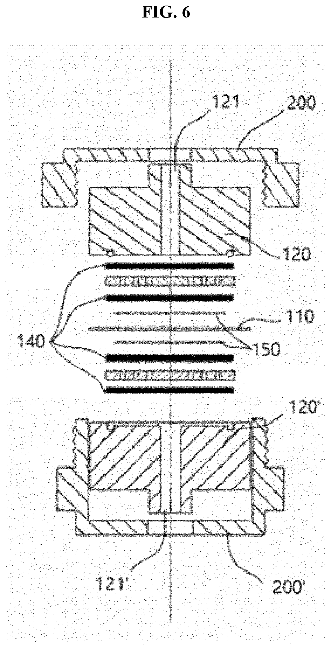

In FIGS. 5 and 6, in a membrane module 100' of the extrusion device 10', between the pair of supports 120 and 120', there are the membrane 110 and the pair of dispersion disks 130 and 130' located in both directions of the membrane 110,

In the membrane module 100' of the extrusion device 10' according to the second embodiment, like the membrane module 100 of the extrusion device 10 according to the first embodiment described above, all of through-holes 121 and 121' of the pair of supports and the centers of the pair of dispersion disks 130 and 130' are located on the same axis. This is to increase the dispersion efficiency of the injected suspension 300, and the principle has been described above, so it will be omitted.

In the membrane module 100', O-rings 140 may be located between the supports 120 and 120' and the dispersion disks 130 and 130' and therefore, since a sealed space is formed so that, when the suspension 300 moves in both directions, a predetermined pressure is applied in a sealed state without leaking the suspension 300 to the outside, the suspension 300 passing through the supports 120 and 120' may be dispersed by the dispersion disks 130 and 130', thereby preventing a membrane clogging phenomenon and further enhancing the increase in usable area of the membrane.

In addition, in the membrane module 100', the O-rings 140 may be located between the membrane 110 and the dispersion disks 130 and 130', thereby forming a sealed space to increase extrusion efficiency when the suspension 300 passes through the membrane.

To prevent damage to the membrane 110, a porous membrane supporting film 150 may be located on one or more surfaces of the membrane 110.

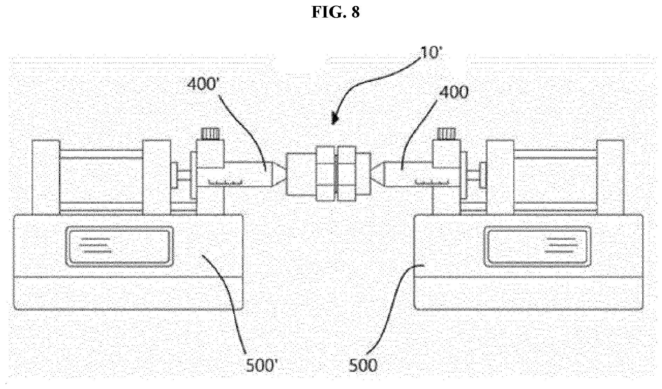

FIG. 8 shows that plungers in first and second syringes 400 and 400' automatically move by syringe pumps 500 and 500', respectively. More preferably, both syringe pumps 500 and 500' may be automatically adjusted to constantly maintain the internal pressures of the first and second syringes 400 and 400' and to this end, a separate pressure gauge (not shown) may be installed.

Hereinafter, a cell extrusion method using the extrusion device 10' according to the second embodiment of the present invention will be described.

First, a first syringe 400 containing a cell-containing suspension 300 is connected to a through-hole 121 at one side of the extrusion device 10', and a second syringe 400' in which a plunger has been pressed is connected to the other through-hole 121' (S'10).

Subsequently, the suspension 300 contained in the first syringe 400 is injected into a membrane module 100' and flows into the second syringe 400' through dispersion disks 130 and 130' and a membrane 110 (S'20).

Subsequently, the suspension 300 flowing into the second syringe 400' may be injected into the membrane module 100' again, and then flow into the first syringe 400 through the dispersion disks 130 and 130' and the membrane 110 (S'30).

S'10 to S'30 have to be repeated at least once, and more preferably, at least 3 to 5 times to obtain a sufficient extrusion effect.

In addition, the present invention includes vesicles prepared by the cell extrusion method. As cells in a suspension are extruded by the extrusion method using the extrusion device according to the present invention, vesicles such as extracellular vesicle mimetics formed by a cell membrane including extracellular vesicles (exosomes, microvesicles, etc.) may be obtained.

The present invention described above is not limited by the embodiments disclosed in the specification, and all forms that can be changed by those of ordinary skill in the art to which the present invention belongs will also be included in the scope of the present invention.

### ^{∗∗∗} Description of reference numerals ^{∗∗∗}

| | | | |
|---|---|---|---|
| 10, 10' : | extrusion device | 100, 100' : | membrane module |
| 110 : | membrane | 120, 120' : | support |
| 121, 121' : | through-hole | 130, 130';: | dispersion disk |
| 131 : | dispersion zone | 132 : | micro hole |
| 140 : | O-ring | 150 : | membrane supporting film |
| 160 : | connection hose | 170 : | container |
| 200, 200' : | holder | 300 : | suspension |
| 400, 400' : | syringe | 500, 500' : | syringe pump |

### [Industrial Applicability]

The present invention relates to a cell extrusion device including a membrane module and a cell extrusion method using the same, in which a membrane clogging phenomenon can be prevented and a usable area of a membrane may be increased by effectively dispersing a cell-containing suspension in the membrane module, and production efficiency can be further increased by replacing a membrane and performing a process easily.

## Claims

1. A cell extrusion device for extruding cells in a suspension passing through a membrane module through constant pressure, wherein the membrane module comprises a pair of supports having a through-hole through which the suspension flows; a membrane through which the cells pass and are extruded; and a dispersion disk having a plurality of micro holes except a dispersing zone having a center, wherein the membrane and the dispersion disk are located between the pair of supports, the through-holes respectively formed in the pair of supports and the center of the dispersion disk are located on the same axis, and the membrane module is closely fixed by a pair of holders.

2. The device of claim 1, wherein the membrane module has an O-ring between the dispersion disk and a support adjacent to the dispersion disk, thereby forming a predetermined space.

3. The device of claim 1, wherein the membrane module has an O-ring between the dispersion disk and the membrane, thereby forming a predetermined space.

4. The device of claim 1, wherein the membrane module has a porous membrane supporting film located on one or more surfaces of the membrane to prevent the membrane from being damaged.

5. A cell extrusion device for extruding cells in a suspension passing through a membrane module through a predetermined pressure, wherein the membrane module comprises a pair of supports having a through-hole through which the suspension flows; a membrane through which the cells pass and are extruded; and a pair of dispersion disks having a plurality of micro holes except a dispersing zone having a center, wherein between the pair of supports, the membrane and the pair of dispersion disks are respectively located in both directions of the membrane, the through-holes respectively formed in the pair of supports and the center of the pair of dispersion disks are located on the same axis, and the membrane module is closely fixed by a pair of holders.

6. The device of claim 5, wherein the membrane module has O-rings between the supports and the dispersion disks, thereby forming a predetermined space.

7. The device of claim 5, wherein the membrane module has O-rings between the membrane and the dispersion disks, thereby forming a predetermined space.

8. The device of claim 5, wherein the membrane module has a porous membrane supporting film on one or more surfaces of the membrane to prevent the membrane from being damaged.

9. The device of claim 1 or 5, wherein, in the membrane module, the syringe containing the suspension is connected to any one or more of the through-holes formed in the pair of supports.

10. The device of claim 9, wherein, in the syringe, a plunger moves by means of a syringe pump.

11. A cell extrusion method using the cell extrusion device of claim 1, comprising:
connecting a syringe having a cell-containing suspension to a through-hole of a support adjacent to the dispersion disk among the through-holes of the cell extrusion device; and injecting the suspension contained in the syringe into the membrane module and discharging the suspension through the other through-hole via the dispersion disk and the membrane, the entire process of which is repeated at least once.

12. A cell extrusion method using the cell extrusion device of claim 5, comprising:
connecting a first syringe including a cell-containing suspension to a through-hole at one side of the cell extrusion device, and connecting a second syringe in which a plunger has been pressed to a through-hole at the other side of the cell extrusion device; injecting the suspension contained in the first syringe into the membrane module to allow the suspension to flow into the second syringe through the dispersion disks and the membrane; and injecting the suspension flowing into the second syringe into the membrane module again to allow the suspension to flow into the first syringe through the dispersion disks and the membrane, and the entire process of which is repeated at least once.

13. Vesicles prepared by the cell extrusion method of claim 11 or 12.
